# EUROPEAN PATENT SPECIFICATION

(11) **EP 4 385 915 B1**
(45) Date of publication and mention of the grant of the patent: **02.12.2026**
(21) Application number: 24172864.1
(22) Date of filing: 19.10.2018
(51) Int. Cl.: A61M 25/00, B65D 43/16, B65D 43/22, B65D 43/24, B65D 53/02, B65D 43/00

(54) **CAPS FOR CATHETER PACKAGES**
KAPPEN FÜR KATHETERVERPACKUNGEN
CAPUCHONS POUR EMBALLAGES DE CATHÉTERS

(30) Priority: 25.10.2017 US 201762577035 P
(43) Date of publication of application: 19.06.2024
(62) Document number(s) of the earlier application(s) in accordance with Art. 76 EPC: 18800400.6 / 3 700 612
(73) Proprietor: Hollister Incorporated, Libertyville, IL 60048 (US)
(72) Inventor: O'BRIEN, Daniel E., Libertyville (US); NAUGHTON, Vincent, Libertyville, IL 60048 (US); CREAVEN, Martin P., Libertyville (US); PUPINO, Scott J., Libertyville, IL 60048 (US); MARCH, Daniel A., Libertyville, IL 60048 (US); HANLEY, Joseph N., Libertyville, IL 60048 (US); LUDLOW, Kevin Anthony, Libertyville, IL 60048 (US); KENDELL, Paige Erin, Libertyville, IL 60048 (US); AMBOURN, Jeffrey A., Libertyville, IL 60048 (US); WOLGEMUTH, David Benjamin, Libertyville, IL 60048 (US); NETT, David Louis, Libertyville, IL 60048 (US); MICHAL, Brandon Lee, Libertyville, IL 60048 (US); BELISLE, Christopher L., Libertyville, IL 60048 (US); PARMER, Timothy Alan, Libertyville, IL 60048 (US)
(74) Representative: FRKelly

(56) References cited:
- WO-A1-2015/066673
- WO-A1-2015/075841
- US-A- 4 113 090
- US-A1- 2016 023 816
- US-A1- 2017 173 300

## Description

### CROSS-REFERENCE TO RELATED APPLICATION

This application claims the benefit of U.S. Provisional Patent Application Serial No. 62/577,035, filed October 25, 2017.

### FIELD OF THE DISCLOSURE

This disclosure relates generally to packaging for medical devices such as urinary catheters. More particularly, this disclosure relates to compact catheters, such as urinary catheters, and the packaging, storing and hydrating/lubricating of such catheters.

### BACKGROUND

Intermittent catheterization is a good option for many users who suffer from a neurogenic bladder, that is, an atonic or unstable bladder associated with a neurological condition, such as diabetes, stroke, or spinal cord injury. Very often a neurogenic bladder is caused by conditions which may also result in diminished dexterity of the user.

Commonly, in intermittent catheterization single use, individually packaged, sterile catheters are used. Catheters often include a surface treatment that reduces friction to allow for easier and less traumatic insertion into and through the user's urethra.

Regardless of whether a surface treatment is used or what type of surface treatment is used, some type of package for the catheter is required. In the past various kinds of packages have been used, including molded containers of assorted sizes and shapes, bags and pouches made of plastic or metal foil, and similar kinds of devices. While these prior art packages generally accomplish the objective of protecting the catheter during transport, storage and preparation for use, they suffer from disadvantages that range from fundamental - the packages may break open prematurely; to economic - the package designs are wasteful of material and labor; to the annoying - the packages confuse users as to how to open them or are difficult for a user of low dexterity to easily access the catheter, or the packages tend to spill the hydrating medium upon opening.

Accordingly, what is needed is a catheter package that is economical to manufacture and fill, reliable throughout its useful life, and simple and intuitive to use. It is also desirable to have a compact package which can be discreetly carried by the user in a purse or pouch; discrete to dispose of in a waste bin; and intuitive and easy to open, particularly by a user with low dexterity. Additional desirable features of the package include easy removal of the catheter from the case; easy reclosing of the case after use; hygienic use; and it should be discreet and clean to carry after use.

Because users often carry intermittent catheters with them in containers such as purses, handbags, shoulder bags, backpacks and the like, the sealed catheter package should be capable of withstanding_compression and other forces to which such containers are typically subjected. More particularly, the catheter package should remain sealed even when subjected to such forces so as to, among other things, maintain the sterility of the catheter within the package.

Furthermore, users will often prefer to return a used catheter to their purse or bag for subsequent disposal. Accordingly, the catheter package should be capable of receiving a used catheter back in the package and then being reclosed in a sealed and secured manner. Thus, a catheter package with a reliably reclosable cap would also be desired. Convenience may be further enhanced if the reclosable cap is attached to the remainder of the package so the cap does not become misplaced during use of the catheter.

As described above, many users of intermittent catheters have limited manual dexterity that can make it difficult for them to open a package and extract a catheter from the package. Thus, while reliable and secure capping and re-capping are a desired aspect of a compact catheter, also desired is the ability to easily open the package, access and extract the catheter. Accordingly, it would be desirable to provide a catheter and catheter package wherein extraction of the catheter is made easier by presenting at least a portion of the catheter (that is not inserted into the urethra of the user, such as the funnel) beyond an open end of the package when the cap is removed. Thus, the end of the funnel is presented for easy extraction and/or for easy and hygienic attachment of a urine collection bag, if desired.

Of course, having at least a portion of catheter or the funnel extending beyond the open end of the package may make the above-described sterile sealing, capping and recapping operations more difficult to achieve. For example, providing a cap hinge that is unobtrusive but affords an arc of motion for the cap that allows the cap to clear the funnel during opening and closing movements and attain the aforementioned sealing (capping) and re-sealing (e.g., "dynamic sealing") presents one challenge. Still another challenge is providing a cap that can be configured to achieve reliable sealing over an extending portion of the catheter (e.g., funnel) while withstanding the forces and loads to which it may be subjected while being carried in a handbag, purse or other receptacle which can compromise the seal. Side loads, i.e., radial or tangential loads on the cap can be a particular problem, especially on the portion of the cap opposite the hinge location. The catheter packages described herein address these concerns. WO2015075841 discloses an intermittent self-catheterization catheter which can be cleaned easily in a short time. US4113090 discloses a package adaptable for use in protecting and maintaining in a sterile condition a number of different types of medical instruments. WO2015066673 discloses a container and cap assembly having a container base with an interior for housing a plurality of products, and a cap affixed to the container base by a hinge, such that the cap pivots with respect to the base to move the assembly between an opened position and a closed position. US2016/023816 discloses a container which includes container body defining an interior and an opening leading to the interior. A lid is movable with respect to the container body to move the container between a closed position in which the lid covers the opening and an opened position in which the opening is exposed.

### SUMMARY

The invention is defined in independent claim 1. Further aspects are defined in dependent claims 2-10.

In one aspect, the present disclosure is directed to a catheter package, including a case having a hollow tube which is closed at one end and open at the other end, and a cap. The package may include a hinge having one end connected to the cap and a second end connected to said other end of the hollow tube. The hinge permits selectable movement of the cap between an open position, wherein access is provided to the open end of the hollow tube, and a closed position, wherein the cap prevents access to the open end of the hollow tube. A seal is connected to at least one of the cap and case. The seal is engageable with the other of the cap and case when the cap is in the closed position to form a barrier between the cap and case that maintains a sterile environment inside the case and cap.

In another aspect, the present disclosure is directed to a catheter package including a case having a hollow tube which is closed at one end and open at the other end and a cap. The package may include a hinge having one end connected to the cap and a second end connected to the other end of the hollow tube. The hinge permits selectable movement of the cap between an open position, wherein access is provided to the open end of the hollow tube, and a closed position, wherein the cap prevents access to the open end of the hollow tube. A seal connected to at least one of the cap and case is provided. The seal is engageable with the other of the cap and case when the cap is in the closed position to form a seal between the cap and case that can be repeatedly made and broken whenever the user closes or opens the cap, respectively.

In a further aspect, the present disclosure is directed to a catheter package that has a hollow plastic case for receiving the catheter. The case has a generally tubular wall closed at one end by a bottom wall. The opposite end of the case is open and has a cap attached to it by a hinge. The cap is selectably movable between open and closed positions in which the cap uncovers or covers the open end of the case, respectively. The cap has a tab extending from the bottom edge of the cap with a hook on the bottom edge of the tab. A depression is formed on the top of the case where it can receive the tab. There is a detent in the depression. When the cap is placed in the closed position the tab fits into the depression and the hook engages the detent to retain the cap in the closed position by releasably resisting vertical lifting forces on the cap. Engagement of the tab in the depression also prevents lateral forces on the closed cap from dislodging the cap or impairing its seal.

In a still further aspect, the present disclosure is directed to a catheter package that has a hollow plastic tube for receiving the catheter. The tube has a generally cylindrical wall closed at one end by a bottom wall. The opposite end of the cylindrical wall has a cylindrical ferrule which is open at its end and defines a rim. At least partially surrounding the ferrule is a collar. At least a portion of the collar is separated from the ferrule, leaving a gap between the ferrule and the collar. The cap is attached by a hinge to the collar. The cap is selectably movable between open and closed positions in which the cap uncovers or covers the open end of the ferrule, respectively. The cap may have a skirt which surrounds the ferrule to cover the ferrule completely. A tongue on the collar is engageable with a groove on the exterior of the cap to retain the cap in the closed position when the cap is pivoted onto the top of the case.

In another aspect, the present disclosure is directed to a catheter package that has a hollow plastic tube for receiving the catheter. The tube has a wall of generally rectangular cross section and is closed at one end by a bottom wall. The opposite end of the tube's wall flares outwardly to an enlarged shoulder with a top land. A cap is attached to the tube by a hinge which allows the cap to move selectably between open and closed positons on the tube. The bottom of the cap has a sealing surface that engages the shoulder when the cap is closed. A seal is placed between the cap and shoulder to seal the interior of the package. Just below the shoulder there is an arcuate rail on the exterior of the tube. A similar rail is located on the exterior of the cap at the cap's lower edge. The cap's rail has a gap in it. A movable slider is engageable with the rails and slidable therealong to alternately lock the closed cap and tube together or unlock the cap for opening when the slider is moved so as to be opposite the gap in the cap rail. Placing the slider in the gap allows opening of the cap.

In another aspect the present disclosure is directed to a catheter package having a hollow plastic case for receiving the catheter. The case has a generally cylindrical wall closed at one end by a bottom wall. The opposite end of the cylindrical wall has a cylindrical ferrule which is open at its end and defines a rim. A cap is attached to the case by a hinge. The cap is made of a relatively stiff material and is engageable with the ferrule to resist side loads. The interior of the cap carries a dome-shaped bell made of a softer material than the cap itself. When the cap is closed the bell is engages the ferrule to seal the case.

In yet another aspect the present disclosure is directed to a catheter package having a hollow plastic case for receiving the catheter and a soft cap for selectably opening and closing the case. The cap has a frame made of a relatively rigid material and which includes an annular ring and an axial spine. The remainder of the cap is attached to the frame and spine and is made of a material softer than the frame.

In a still further aspect, the present disclosure is directed to a catheter package that has a hollow plastic case for receiving the catheter. One end of the case has a cylindrical ferrule which is open at its end and defines a rim. At least partially surrounding the ferrule is a collar. At least a portion of the collar is separated from the ferrule, leaving a gap between the ferrule and the collar. A cap is attached by a hinge to the collar. The cap is selectably movable between open and closed positions in which the cap uncovers or covers the open end of the ferrule, respectively. A soft liner on the interior of the cap engages the ferrule when the cap is closed to seal the ferrule. Latch fingers extend radially from the ferrule. The cap may have a skirt which has an opening for receiving the latch fingers to releasably retain the cap in a closed position.

In an additional aspect, the present disclosure is directed to a catheter package that has a hollow plastic case for receiving the catheter. The case is molded with an open top that is sealed with a separate cover. The cover can be a heat sealed foil or the like. The package can be opened by removing the cover. Alternately, the package can be opened by breaking through a molded-in, thin wall section of the case near the top.

In a further additional aspect, the present disclosure is directed to a catheter package that has a hollow plastic case for receiving the catheter. The case has a generally tubular wall closed at one end by a bottom wall. The opposite end of the case is open and has a cap attached to it by a hinge. The cap is selectably movable between open and closed positions in which the cap uncovers or covers the open end of the case, respectively. A locking arm is pivotably connected to the case. The locking arm can pivot upwardly to engage a detent on the cap when the cap is closed and thereby retain the cap in the closed position.

Most of the packages of the present disclosure permit a user to retrieve the catheter from the case and re-capture it if they so wish. Once the cap is locked back into its closed position the package retains its original sealing qualities (meaning it will not leak), with a feature, such as a label that breaks upon opening, indicating that the product has been used. Another potential indication of use could be stress marks created in the hinge when the user first opens the product.

### BRIEF DESCRIPTION OF THE DRAWINGS

Fig. 1 is a perspective view of a first embodiment of the open or upper end portion of the catheter package of the present disclosure, with the cap in the fully open position.
Fig. 2 is a perspective view of the package of Fig. 1, with the cap shown in a partially open position.
Fig. 3 is a perspective view of the package of Fig. 1, with the cap in the fully closed position.
Fig. 4 is a section taken along line 4-4 of Fig. 2.
Fig. 5 is a section taken along line 5-5 of Fig. 3.
Fig. 6 is a perspective view of a second embodiment of the package of the present disclosure, with the cap in the fully closed position.
Fig. 7 is a section taken along line 7-7 of Fig. 6.
Fig. 8 is a perspective view of the section of Fig. 7.
Fig. 9 is a perspective view of the inside of the cap of the second embodiment.
Fig. 10 is a perspective view of a third embodiment of the catheter package of the present disclosure, with the cap in the fully closed position.
Fig. 11 is a perspective view of the package of Fig. 10 viewed from a different angle and with the locking slide moved to the open position.
Fig. 12 is a view similar to Fig. 10 but with the cap and locking slide in the fully open position.
Fig. 13 is a section taken along line 13-13 of Fig. 10.
Fig. 14 is a perspective view of a fourth embodiment of the catheter package of the present disclosure, with the cap in the partially open position.
Fig. 15 is a view similar to Fig. 14 but with the cap fully open.
Fig. 16 is a section taken along line 16-16 of Fig. 15.
Fig. 17 is a longitudinal section of the package of Fig. 14, on an enlarged scale.
Fig. 18 is a section, similar to Fig. 16 but showing the cap partially open.
Fig. 19 is a perspective view of a fifth embodiment of the catheter package of the present disclosure, with the cap in a fully open position.
Fig. 20 is an exploded side elevation view of the package of Fig. 19.
Fig. 21 is a perspective view of a sixth embodiment of the catheter package of the present disclosure, with the cap in a partially open position.
Fig. 22 is a front elevation view of the package of Fig. 21 with the cap in the closed position.
Fig. 23 is an exploded perspective view of a seventh embodiment of the catheter package of the present disclosure.
Fig. 24 is a perspective view of an eighth embodiment of the catheter package of the present disclosure.

### DETAILED DESCRIPTION OF THE EMBODIMENTS

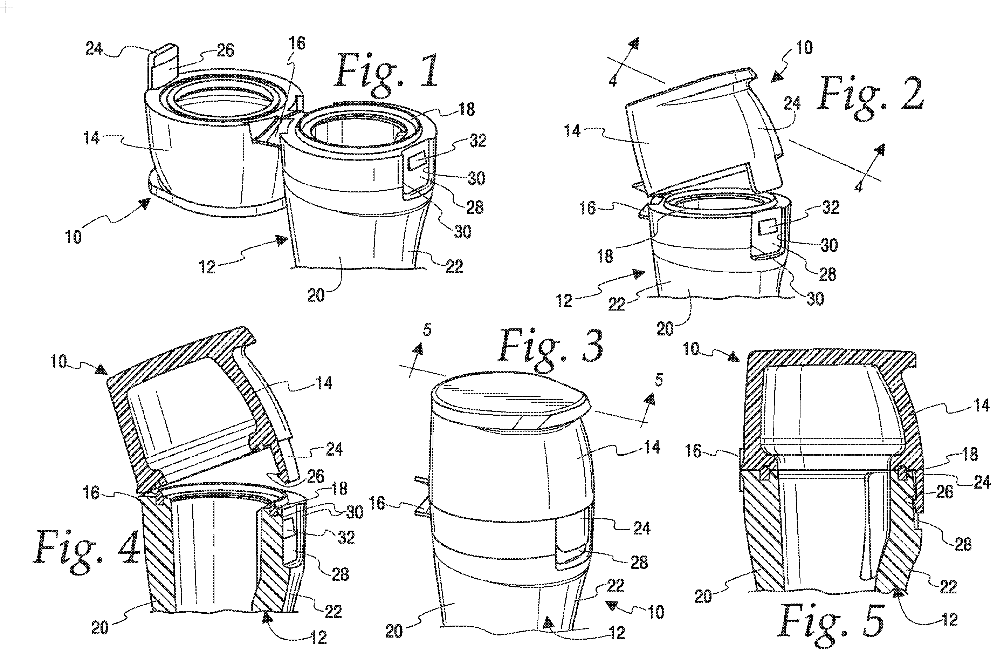

The present disclosure is directed to packages for medical devices such as intermittent urinary catheters. A first embodiment of such a package is shown generally at 10 in Figs. 1-5. The major components of the package include a case 12, a cap 14, a hinge 16 connecting the cap 14 and case 12, and a seal ring 18. The case is preferably molded from a suitable plastic material, such as polypropylene, although other materials could be used. The case includes a hollow tubular wall 20. Only the open or upper end portion of the tubular wall is shown. The lower end is broken away. The tubular wall 20 terminates at its lower end at a transverse end wall (not shown) that closes the bottom of the tubular wall. The interior surface of the tubular wall may be generally cylindrical. The exterior surface of the tubular wall 20 may have either a cylindrical or rectangular cross-sectional shape or the cross-section could be otherwise. The upper end portion of the tubular wall 20 flares outwardly somewhat at a neck portion 22 to increase the diameter of the tube. The top of the neck 22 terminates at an open end of the tubular wall 20.

The hinge 16 as shown is a living hinge, but other hinge arrangements could be used. The hinge connects to the case 12 and the cap 14 to permit selectable movement of the cap between open and closed positions. The cap 14 is selectably movable between the open position shown in Figs. 1, 2 and 4 and the closed position shown in Figs. 3 and 5. The cap uncovers or covers the open end of the case in the open and closed positions, respectively.

The cap has a tab 24 extending from the bottom edge of the cap. There is an inwardly directed hook 26 on the bottom edge of the tab 24. A depression 28 is formed on the top of the case 12 and defines side edges 30. The depression is aligned with the tab 24 when the cap is closed. There is a detent 32 formed in the depression 28. When the cap is placed in the closed position the tab 24 fits into the depression 28 as seen in Figs. 3 and 5. The tab flexes to allow the hook 26 to slide past the detent 32 and engage the detent to retain the cap 14 in the closed position by releasably resisting vertical lifting forces on the cap. Since the closed tab 24 is also immediately adjacent the sides edges 32 defined by the depression 28, the tab also prevents lateral forces on the closed cap from dislodging the cap or impairing its seal. The seal is formed when the cap is closed by engagement of the bottom land of the cap with the upwardly protruding portion of the seal ring 18. This seal is dynamic in that it can be made and released multiple times as the cap is closed or opened.

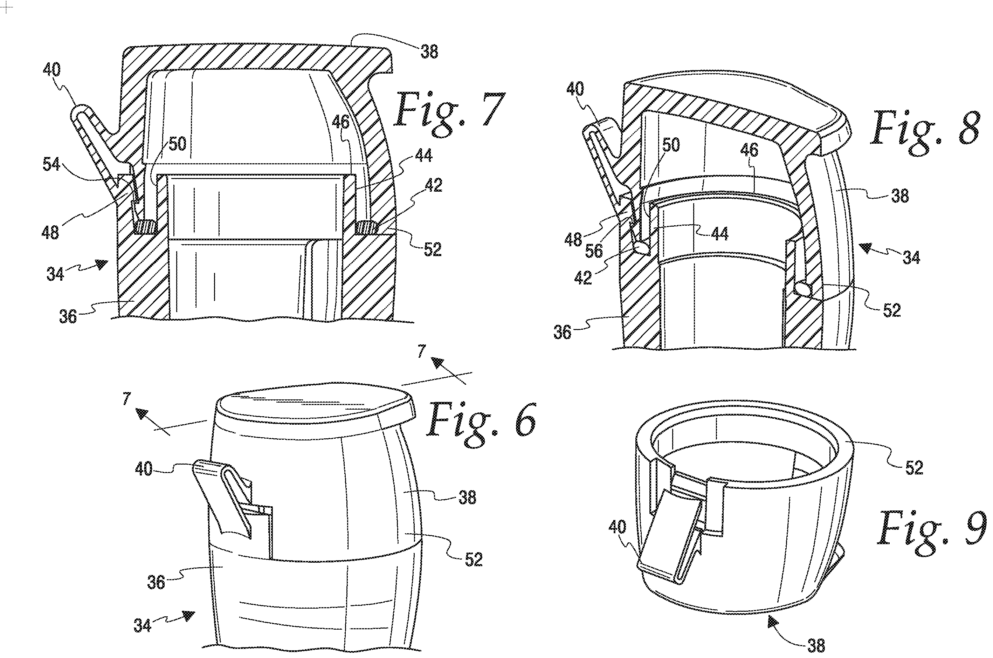

A second embodiment of a package for medical devices such as intermittent urinary catheters is shown generally at 34 in Figs. 6-9. The major components of the package once again include a case 36, a cap 38, a hinge 40 connecting the cap 38 and case 36, and a seal ring 42. As in the previous embodiment, the case 36 is a generally hollow plastic tube for receiving the catheter. The tube has a generally cylindrical wall closed at one end by a bottom wall (not shown). The opposite end of the cylindrical wall has a cylindrical ferrule 44 which is open at its end and defines a rim 46. At least partially surrounding the ferrule is a collar 48. At least a portion of the collar 48 (on the left side of Figs. 7 and 8) is separated from the ferrule 44, leaving a gap 50 between the ferrule 44 and the collar 48. The cap 38 is attached by the U-shaped hinge 40 to the collar 48. The cap is selectably movable between open and closed positions in which the cap 38 uncovers or covers the open end of the ferrule 44, respectively. The cap may have a skirt 52 which surrounds the ferrule 44 to cover it completely when the cap is closed. Also, when the cap is closed a portion of the skirt 52 fits within the gap 50 and the skirt 52 engages the seal ring 42. A tongue 54 on the interior of the collar 48 is engageable with a groove 56 on the exterior of the cap's skirt 52 to retain the cap 38 in the closed position when the cap is pivoted onto the top of the case.

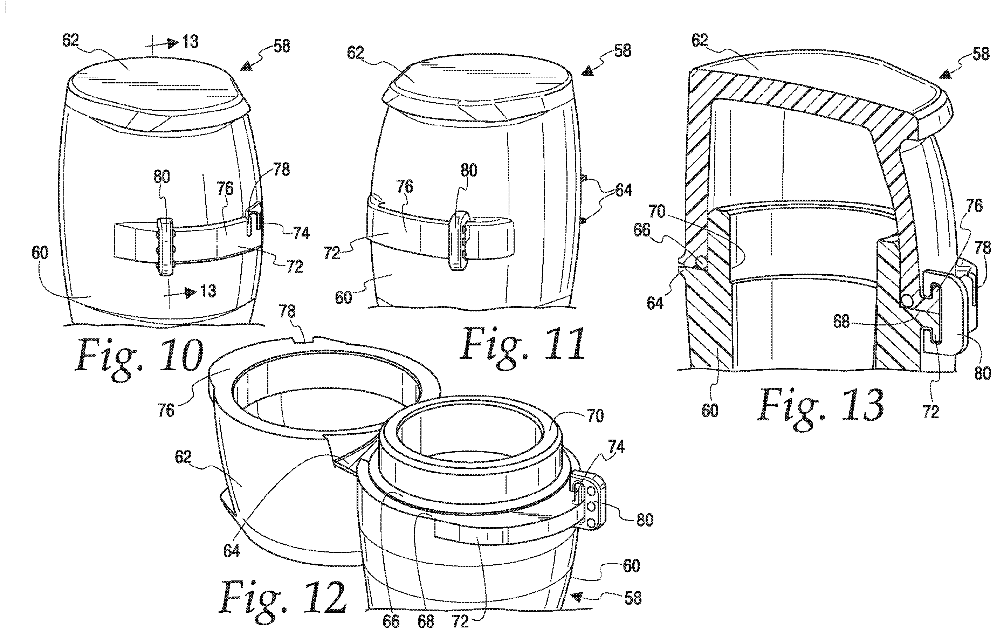

A third embodiment of a package for urinary catheters is shown generally at 58 in Figs. 10-13. As in the previous embodiments the major components of the package include a case 60, a cap 62, a hinge 64 connecting the cap 62 and case 60, and a seal ring 66. The case 60 once again is a tube with a cylindrical wall closed at one end by a bottom wall (not shown). The opposite end of the tube's wall has a radial shoulder 68 with a ferrule 70 extending axially above the shoulder. The seal ring 66 rests on top of the shoulder 68. The bottom of the cap 62 has a sealing surface that engages the shoulder 68 and the seal ring 66 when the cap is closed. Just below the shoulder 68 there is an arcuate rail 72 on the exterior of the case. There is a retaining post 74 that extends above the shoulder 68 but is attached to the shoulder. Another rail 76 is located on the exterior of the cap at the cap's lower edge. The cap's rail 76 has a gap 78 in it that receives the retaining post 74 when the cap is closed. A movable, C-shaped locking slide 80 has hooks or fingers that are engageable with the rails 72 and 74. The locking slide is arcuately movable along the rails to alternately lock the closed cap and tube together or unlock the cap for opening. The locking slide is always engaged with the case rail 72. It is alternately engaged with either the cap rail 74 or the retaining post 74. When the slide 80 is moved so as to be opposite the gap 78 in the cap rail 76 the slide engages the retaining post 74 and releases the cap. Placing the slide opposite the gap 76 disengages the slide from the cap rail 74 to permit opening of the cap. Reclosing the cap places the gap 78 around the slide 80 and allows the slide to be moved laterally back into engagement with the cap rail 76, thereby locking the cap to the case.

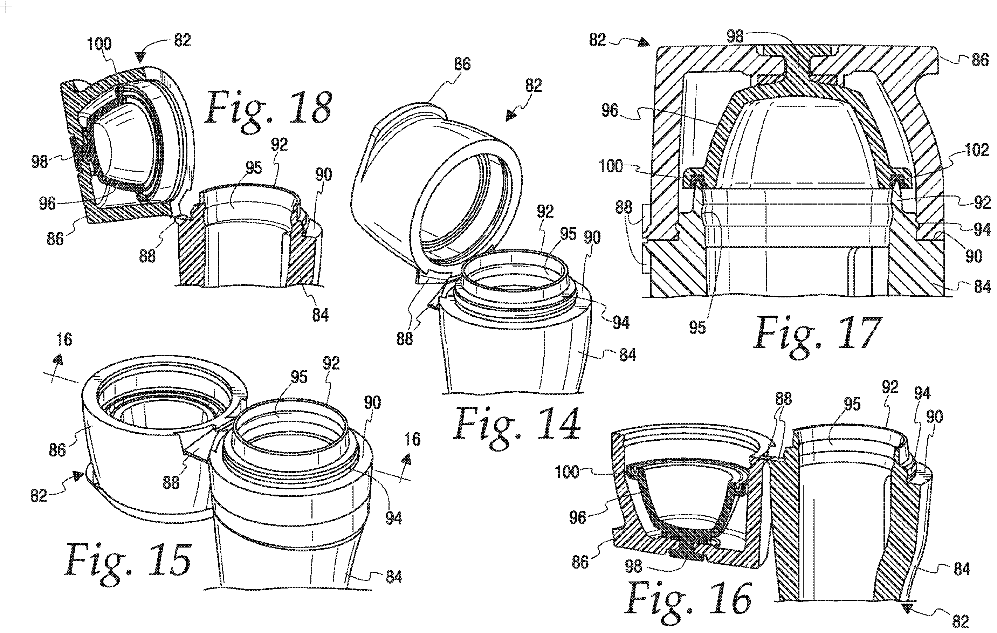

The invention is shown in the fourth embodiment of a package for urinary catheters shown generally at 82 in Figs. 14-18. As in the previous embodiments the major components of the package include a case 84, a cap 86, and a hinge 88 connecting the cap 86 and case 84. The case has a generally cylindrical wall closed at one end by a bottom wall (not shown). The opposite end of the cylindrical wall has a radial shoulder 90 with a ferrule 92 extending axially above the shoulder. There is an enlargement at the junction of the shoulder and ferrule. An outwardly-protruding, radial bead 94 is formed on the enlargement. The bead is engageable with a groove 95 in the interior of the cap when the cap is closed. The bead and cap are made of relatively stiff materials so as to resist side loads on the cap. The interior of the cap carries a dome-shaped bell 96 made of a softer material than the cap itself. The bell is connected to the cap by a post 98. The lower edge of the bell has a flange sized to engage the top of the ferrule 92 when the cap is closed to seal the case. The soft material of the bell affords a tight seal on the harder ferrule. Alternately, a seal ring 102 could be carried on the underside of the flange 100.

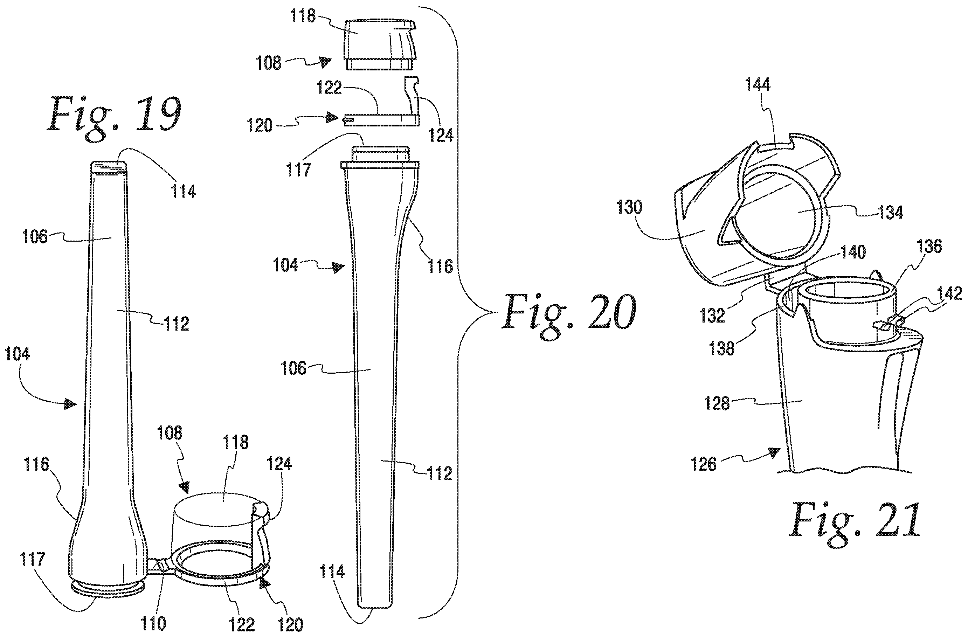

A fifth embodiment of a package for urinary catheters is shown generally at 104 in Figs. 19-20. This package includes a case 106, a cap 108, and a hinge 110 connecting the cap 108 to the case 106. The case includes a hollow tubular wall 112. The tubular wall 112 terminates at its lower end at a transverse end wall 114 that closes the bottom of the tubular wall. The upper end portion of the tubular wall 112 flares outwardly somewhat at a neck portion 116 to increase the diameter of the tube. The top of the neck 116 terminates at an open end 117 of the tubular wall 112. The hinge 110 permits selectable movement of the cap 108 between open and closed positions. The cap has a soft shell 118 supported by a frame 120 made of a relatively rigid material. The frame has an annular ring 122 and an axial spine 124. The soft shell 118 of the cap 108 is attached to the annular ring 122 and the spine 124 and is made of a material that is softer than these parts of the frame 120.

A sixth embodiment of a package for urinary catheters is shown generally at 126 in Figs. 21-22. The package has a case 128, a cap 130, a hinge 132 connecting the cap 130 and case 128, and a seal liner 134 in the interior of the cap. The case 128 is a generally hollow tube having a wall closed at one end by a bottom wall (not shown). The opposite end of the cylindrical wall is similar to that of Figs. 6-9 in that it has a cylindrical ferrule 136 which is open at its end. At least partially surrounding the ferrule 136 is a collar 138. At least a portion of the collar 138 is separated from the ferrule 136, leaving a gap 140 between the ferrule 136 and the collar 138. The cap 130 is attached by the hinge 132 to the collar 138. The cap is selectably movable between open and closed positions in which the cap 130 uncovers or covers the open end of the ferrule 136, respectively. The soft liner 134 on the interior of the cap 130 engages the ferrule 136 when the cap is closed to seal the ferrule. Latch fingers 142 extend radially from the ferrule 136. The cap 130 may have a skirt which has an opening 144 for receiving the latch fingers 142 to releasably retain the cap in a closed position.

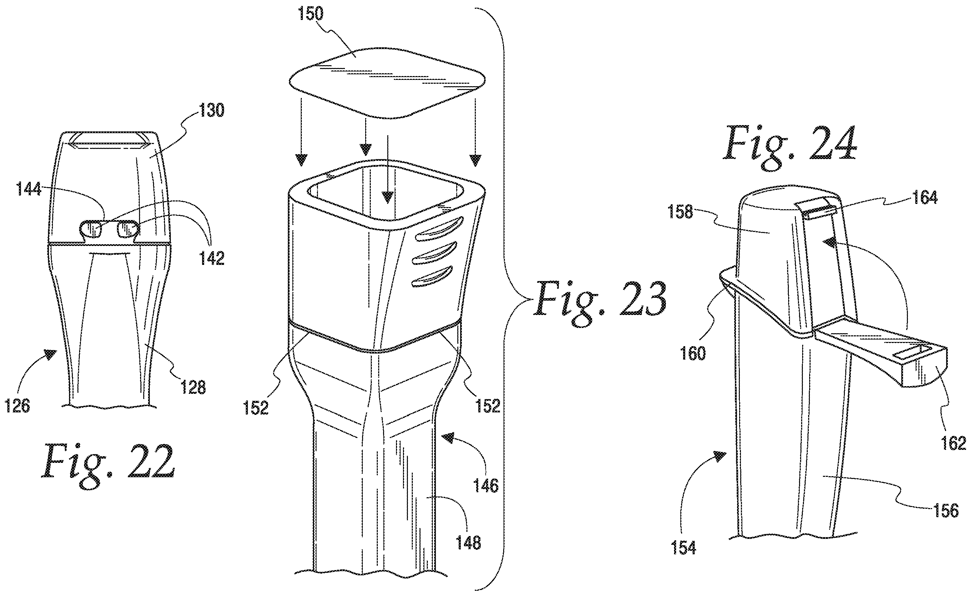

A seventh embodiment of a package for urinary catheters is shown generally at 146 in Fig. 23. Package 146 includes a case 148 with an open top that is sealed with a separate cover 150. The cover can be a foil layer or the like that is heat sealed or fixed to the case 148 by an adhesive. Toward the top end of the case there is a thin-walled portion 152 on at least three sides of the case. The thin-walled portion 152 has a reduced wall thickness compared to the rest of the case. This affords a weakened connection that allows the package to be opened by breaking through the molded-in, thin-walled section 152 of the case. Alternately, the package 146 can be opened by removing or piercing the cover 150.

An eighth embodiment of a package for urinary catheters is shown generally at 154 in Fig. 24. The package has a case 156, a cap 158, a hinge 160 connecting the cap 158 and case 156. The case has a generally tubular wall closed at one end by a bottom wall. The opposite end of the case is open and has the cap attached to it by the hinge 160. The cap 158 is selectably movable between open and closed positions in which the cap uncovers or covers the open end of the case, respectively. A locking arm 162 is pivotably connected to the case 156. The locking arm 162 can pivot upwardly to engage a detent 164 on the cap 158 when the cap is closed and thereby retain the cap in the closed position.

It should be understood that various changes and modifications to the presently preferred embodiments described herein will be apparent to those skilled in the art. Such changes and modification can be made without departing from the scope of the invention disclosed herein.

## Claims

1. A catheter package (82), comprising:
a case (84) having a hollow tube which is closed at one end and open at the other end;
a cap (86); and
a hinge (88) having one end connected to the cap and a second end connected to said other end of the hollow tube, the hinge permitting selectable movement of the cap between an open position, wherein access is provided to the open end of the hollow tube, and a closed position, wherein the cap prevents access to the open end of the hollow tube;
wherein the other end of the case includes a radial shoulder (90) with a ferrule (92) extending axially above the radial shoulder with an enlargement at a junction of the radial shoulder and the ferrule; and
wherein a radial bead (94) is on the enlargement.

2. The catheter package of claim 1, wherein the cap includes a groove (95) in an interior of the cap.

3. The catheter package of claim 2, wherein the radial bead is engageable with the groove when the cap is in the closed position.

4. The catheter package of claim 1, wherein the radial bead and the cap are comprised of relatively stiff materials.

5. The catheter package of claim 1, wherein the open end of the case has a first portion and a second portion and the catheter package further comprising:
a bell (96) mounted on the interior of the cap, the cap and the radial bead being made of a relatively stiff material compared to that of the bell and resist side loads on the cap, a lower edge of the bell includes a flange sized to engage a top of the ferrule when the cap is closed to seal the case.

6. The catheter package of claim 5 wherein the bell is connected to the cap by a post (98).

7. The catheter package of claim 5, wherein the bell is dome shaped.

8. The catheter package of claim 5, wherein a lower edge of the bell has a flange (100) sized to engage a top of the ferrule when the cap is in a closed position.

9. The catheter package of claim 8, wherein the bell is comprised of a softer material and the ferrule is comprised of a harder material.

10. The catheter package of claim 8, further comprising a seal ring (102) on an underside of the flange.

## Patentansprüche

1. Katheterverpackung (82), umfassend:
ein Gehäuse (84) mit einem Hohlrohr, das an einem Ende geschlossen und am anderen Ende offen ist;
eine Kappe (86); und
ein Scharnier (88), dessen eines Ende mit der Kappe und dessen zweites Ende mit dem genannten anderen Ende des Hohlrohrs verbunden ist, wobei das Scharnier eine wählbare Bewegung der Kappe zwischen einer offenen Stellung, in der ein Zugang zu dem offenen Ende des Hohlrohrs bereitgestellt ist, und einer geschlossenen Stellung, in der die Kappe einen Zugang zu dem offenen Ende des Hohlrohrs verhindert, ermöglicht;
wobei das andere Ende des Gehäuses eine radiale Schulter (90) einschließt, mit einer Hülse (92), die sich axial über die radiale Schulter hinaus erstreckt, mit einer Verbreiterung an einer Verbindungsstelle der radialen Schulter und der Hülse; und
wobei sich eine radiale Wulst (94) auf der Verbreiterung befindet.

2. Katheterverpackung nach Anspruch 1, wobei die Kappe eine Nut (95) in einem Inneren der Kappe einschließt.

3. Katheterverpackung nach Anspruch 2, wobei die radiale Wulst mit der Nut in Eingriff bringbar ist, wenn sich die Kappe in der geschlossenen Stellung befindet.

4. Katheterverpackung nach Anspruch 1, wobei die radiale Wulst und die Kappe aus relativ steifen Materialien bestehen.

5. Katheterverpackung nach Anspruch 1, wobei das offene Ende des Gehäuses einen ersten Abschnitt und einen zweiten Abschnitt aufweist und die Katheterverpackung ferner Folgendes umfasst: eine Glocke (96), die an der Innenseite der Kappe angebracht ist, wobei die Kappe und die radiale Wulst aus einem im Vergleich zu dem der Glocke relativ steifen Material bestehen und seitlichen Belastungen der Kappe widerstehen, wobei eine Unterkante der Glocke einen Flansch einschließt, der so bemessen ist, dass er in eine Oberseite der Hülse eingreift, wenn die Kappe geschlossen ist, um das Gehäuse abzudichten.

6. Katheterverpackung nach Anspruch 5, wobei die Glocke über einen Stift (98) mit der Kappe verbunden ist.

7. Katheterverpackung nach Anspruch 5, wobei die Glocke kuppelförmig ist.

8. Katheterverpackung nach Anspruch 5, wobei eine Unterkante der Glocke einen Flansch (100) aufweist, der so bemessen ist, dass er in eine Oberseite der Hülse eingreift, wenn sich die Kappe in einer geschlossenen Stellung befindet.

9. Katheterverpackung nach Anspruch 8, wobei die Glocke aus einem weicheren Material und die Hülse aus einem härteren Material besteht.

10. Katheterverpackung nach Anspruch 8, ferner umfassend einen Dichtungsring (102) an einer Unterseite des Flansches.

## Revendications

1. Emballage de cathéter (82), comprenant :
un étui (84) ayant un tube creux qui est fermé à une extrémité et ouvert à l'autre extrémité ;
un capuchon (86) ; et
une charnière (88) ayant une extrémité reliée au capuchon et une seconde extrémité reliée à ladite autre extrémité du tube creux, la charnière permettant un mouvement sélectionnable du capuchon entre une position ouverte, dans lequel l'accès est prévu à l'extrémité ouverte du tube creux, et une position fermée, dans lequel le capuchon empêche l'accès à l'extrémité ouverte du tube creux ;
dans lequel l'autre extrémité de l'étui comporte un épaulement radial (90) avec une virole (92) s'étendant axialement au-dessus de l'épaulement radial avec un élargissement à une jonction de l'épaulement radial et de la virole ; et
dans lequel un bourrelet radial (94) est situé sur l'élargissement.

2. Emballage de cathéter selon la revendication 1, dans lequel le capuchon comporte une rainure (95) à l'intérieur du capuchon.

3. Emballage de cathéter selon la revendication 2, dans lequel le bourrelet radial peut venir en prise dans la rainure lorsque le capuchon est en position fermée.

4. Emballage de cathéter selon la revendication 1, dans lequel le bourrelet radial et le capuchon sont composés de matériaux relativement rigides.

5. Emballage de cathéter selon la revendication 1, dans lequel l'extrémité ouverte de l'étui a une première partie et une seconde partie, et l'emballage de cathéter comprenant en outre : une cloche (96) montée à l'intérieur du capuchon, le capuchon et le bourrelet radial étant faits d'un matériau relativement rigide par rapport à celui de la cloche et résistant aux charges latérales sur le capuchon, un bord inférieur de la cloche comporte une bride dimensionnée pour venir en prise sur le haut de la virole lorsque le capuchon est fermé pour sceller l'étui.

6. Emballage de cathéter selon la revendication 5, dans lequel la cloche est reliée au capuchon par un plot (98).

7. Emballage de cathéter selon la revendication 5, dans lequel la cloche est en forme de dôme.

8. Emballage de cathéter selon la revendication 5, dans lequel un bord inférieur de la cloche a une bride (100) dimensionnée pour venir en prise avec le haut de la virole lorsque le capuchon est en position fermée.

9. Emballage de cathéter selon la revendication 8, dans lequel la cloche est composée d'un matériau plus souple et la virole est composée d'un matériau plus dur.

10. Emballage de cathéter selon la revendication 8, comprenant en outre un anneau d'étanchéité (102) situé sur une face inférieure de la bride.
